Europäisches Patentamt

European Patent Office

Office européen des brevets

(19)

(11) Veröffentlichungsnummer : **0 351 616 B1**

(12)                    **EUROPÄISCHE PATENTSCHRIFT**

(45) Veröffentlichungstag der Patentschrift :
**16.09.92 Patentblatt 92/38**

(51) Int. Cl.$^5$ : **C07C 69/533**, C07C 67/38

(21) Anmeldenummer : **89112039.6**

(22) Anmeldetag : **01.07.89**

(54) **Verfahren zur Herstellung von Pentensäurealkylestern.**

(30) Priorität : **22.07.88 DE 3824958**

(43) Veröffentlichungstag der Anmeldung :
**24.01.90 Patentblatt 90/04**

(45) Bekanntmachung des Hinweises auf die
Patenterteilung :
**16.09.92 Patentblatt 92/38**

(84) Benannte Vertragsstaaten :
**BE CH DE ES FR GB IT LI NL**

(56) Entgegenhaltungen :
**EP-A- 0 267 497**
**EP-A- 0 268 864**
**FR-A- 1 402 383**
**US-A- 3 661 948**

(72) Erfinder : **Maerkl, Robert, Dr.**
**Bolander Weg 23**
**W-6701 Fussgoenheim (DE)**
Erfinder : **Bertleff, Werner, Dr.**
**Franz-Marc-Strasse 12**
**W-6806 Viernheim (DE)**
Erfinder : **Schuch, Gunter, Dr.**
**Ulrich-v.-Hutten-Strasse 5**
**W-6700 Ludwigshafen (DE)**
Erfinder : **Stops, Peter**
**Limburgstrasse 12**
**W-6701 Altrip (DE)**
Erfinder : **Kuehn, Gebhard**
**Am Weidenschlag 92**
**W-6700 Ludwigshafen (DE)**
Erfinder : **Panitz, Paul**
**Wachenheimer Strasse 1**
**W-6520 Worms 1 (DE)**

(73) Patentinhaber : **BASF Aktiengesellschaft**
**Carl-Bosch-Strasse 38**
**W-6700 Ludwigshafen (DE)**

EP 0 351 616 B1

**Beschreibung**

Aus der US-PS 4 550 195 ist ein Verfahren zur Herstellung von Pentensäurealkylestern bekannt, bei dem man Butadien enthaltende $C_4$-Schnitte mit Kohlenmonoxid und Alkanolen in Gegenwart von Cobaltcarbonylkatalysatoren und heterocyclischen aromatischen tertiären Stickstoffbasen umsetzt. Bei der technischen Durchführung des Verfahrens hat sich herausgestellt, daß die Raum-Zeit-Ausbeute zu wünschen übrig läßt, insbesondere dann, wenn das im Überschuß angewandte Kohlenmonoxid im Kreis geführt und wieder verwendet wird.

Es war deshalb die technische Aufgabe gestellt, bei der Herstellung von Pentensäurealkylestern durch Carbalkoxylierung von Butadien die Selektivität zu Pentensäurealkylestern und die Raum-Zeit-Ausbeute zu steigern, ohne daß der Anfall an Nebenprodukten erhöht wird.

Diese Aufgabe wird gelöst in einem Verfahren zur Herstellung von Pentensäurealkylestern durch Umsetzen von Butadien mit Kohlenmonoxid und Alkanolen in Gegenwart von Cobaltcarbonylkomplexen und tertiären aromatischen heterocyclischen Stickstoffbasen bei einer Temperatur von 80 bis 160°C und unter einem Druck von 100 bis 1000 bar, wobei man 0,1 bis 10 mol-% Wasser, bezogen auf eingesetztes Butadien mitverwendet.

Das neue Verfahren hat den Vorteil, daß es mit hoher Selektivität und hoher Raum-Zeit-Ausbeute verläuft, ohne daß erhöhte Mengen an Nebenprodukten gebildet werden.

Als Ausgangsverbindung verwendet man Butadien. Geeignet sind auch Butadien enthaltende Kohlenwasserstoffgemische, insbesondere Butadien enthaltende $C_4$-Schnitte. Solche $C_4$-Schnitte enthalten z.B. durchschnittlich 40 bis 80 Gew.-% Butadien, 20 bis 35 Gew.-% Isobuten, 10 bis 25 Gew.-% Buten-1, 2 bis 15 Gew.-% Buten-2 soweie 1 bis 10 Gew.-% Butan.

Geeignete Alkanole haben vorteilhaft 1 bis 6 Kohlenstoffatome, insbesondere 1 bis 4 Kohlenstoffatome. Beispielhaft seien genannt: Methanol, Ethanol, Isopropanol, Butanol oder Hexanol. Besonders bevorzugt ist Methanol.

Die Alkanole wendet man in der Regel im Überschuß an. Vorteilhaft verwendet man je Mol Butadien 1,1 bis 10 Mol, insbesondere 1,1 bis 5 Mol Alkanole.

Die Umsetzung wird bei einer Temperatur von 80 bis 160°C durchgeführt. Besonders bewährt hat sich eine Temperatur von 100 bis 150°C. Ferner hält man bei der Umsetzung einen Druck von 100 bis 1000 bar, insbesondere von 120 bis 700 bar ein.

Kohlenmonoxid wird vorteilhaft im Überschuß, z.B. dem 1,3- bis 10-fachen der stöchiometrisch erforderlichen Menge angewandt. Sofern das Verfahren kontinuierlich durchgeführt wird, führt man überschüssiges Kohlenmonoxid fortwährend im Kreis und ergänzt es mit frischem Kohlenmonoxid.

Die verwendeten Kobaltcarbonylkatalysatoren werden entweder in situ aus Cobaltsalzen, z.B. fettsauren Cobaltsalzen wie Cobaltformiat, -acetat, -propionat oder -butyrat erzeugt. Vorteilhaft bringt man den Katalysator bereits als Cobaltcarbonyl ein. Insbesondere hat es sich bewährt, wenn man Kobaltkatalysatoren gelöst in Butadien oder $C_4$-Schnitt in das Reaktionsgemisch einbringt. Eine solche Lösung erhält man beispielsweise durch Umsetzen einer wäßrigen Lösung von fettsauren Cobaltsalzen mit einem Gemisch aus Kohlenmonoxid und Wasserstoff in Gegenwart von Aktivkohle bei einer Temperatur von 100 bis 160°C und unter einem Druck von 100 bis 400 bar. Aus der wäßrigen Lösung werden die entstandenen Cobaltcarbonylverbindungen dann mit Butadien oder $C_4$-Schnitt extrahiert.

Die Umsetzung wird in Gegenwart von heterocyclischen, aromatischen tertiären Stickstoffbasen vorteilhaft mit einem $pK_a$-Wert von 6 bis 9 durchgeführt. Geeignete Stickstoffbasen sind beispielsweise 3-Methylpyridin ($pK_a$ 6,0), 4-Methyl- oder 3,5-Dimethylpyridin ($PK_a$ 6,2), ferner Isochinolin. Besondere technische Bedeutung haben 3-Methylpyridin und 4-Methylpyridin erlangt. Es ist auch möglich, Gemische der genannten Stickstoffbasen anzuwenden. Besonders bewährt hat es sich, wenn man je Mol Cobaltkatalysator 2 bis 25 Mol der vorgenannten Stickstoffbasen anwendet.

Je Mol Butadien verwendet man vorteilhaft 0,01 bis 0,25 Mol Cobaltkatalysator, insbesondere 0,04 bis 0,2 Mol Cobaltkatalysator.

Erfindungsgemäß werden, bezogen auf die eingesetzte Menge an Butadien 0,1 bis 10 mol-%, insbesondere 0,5 bis 8 mol-% Wasser mitverwendet. In der Regel wird das Wasser gemeinsam mit Ausgangsstoffen der Reaktion zugeführt.

Vorteilhaft wird die Umsetzung kontinuierlich, z.B. in mehreren hintereinander geschalteten Stufen, z.B. 2 bis 4 Stufen, insbesondere Schlaufenreaktoren durchgeführt. In einer vorteilhaften Ausführungsform wird das Wasser, z.B. in der ersten und/oder den nachfolgenden Stufen, z.B. der zweiten Stufe, zugegeben.

Das Verfahren nach der Erfindung führt man beispielsweise durch, indem man zwei hintereinander geschaltete Hochdruckgefäße aus nicht-rostendem Stahl, z.B. zwei hintereinander geschaltete Schlaufenreaktoren verwendet und in die erste Stufe fortlaufend Butadien enthaltenden $C_4$-Schnitt, Alkanole, Cobaltcarbonylkatalysatoren, heterocyclische, aromatische tertiäre Stickstoffbasen, Wasser und Kohlenmonoxid in den angegebenen Verhältnissen zuführt und das Reaktionsgemisch auf der angegebenen Temperatur und dem angegebenen Druck hält. In dem Maße wie Ausgangsstoffe zugeführt werden, wird in der zweiten Stufe Reaktionsgemisch entnommen und das Kohlenmonoxid abgetrennt und wiederum mit frischem Kohlenmonoxid dem Ausgangsgemisch zugesetzt. Das verbleibende Reaktionsgemisch wird nach Entspannen, z.B. durch Destillation von überschüssigen Kohlenwasserstoffen befreit und Pentensäurealkylester abdestilliert und der Cobaltkatalysator zurückgeführt. Nach einer anderen Arbeitsweise wird der Cobaltkatalysator, z.B. durch Behandeln in wäßrig-saurem Medium mit molekularem Sauerstoff enthaltenden Gasen und Abtrennen der Cobaltsalzlösung entfernt und die organische Phase durch Destillation aufgearbeitet.

Nach dem Verfahren der Erfindung erhält man vornehmlich 3-Pentensäureester mit wechselnden Mengen an 4- und 2-Pentensäureestern, die sich zur Herstellung von Adipinsäureestern eignen.

Das Verfahren nach der Erfindung sei an folgenden Beispielen veranschaulicht. Die angegebenen Gewichtsteile verhalten sich zu den Volumenteilen wie Kilogramm zu Liter.

Beispiel 1

Einem Hochdruckgefäß von 260 Vol.-Teilen Inhalt führt man von unten im Verlauf von 60 Minuten 26 Gew.-Teile $C_4$-Schnitt mit 41 % (m/m) Butadien-1,3, 22 Gew.-Teile 3-Methylpyridin, 8,8 Gew.-Teile Methanol, 0,8 Gew.-Teile Cobalt in Form von Cobaltcarbonyl 0,14 Gew.-Teile Wasser und 22 Gew.-Teile eines Gasgemisches folgender Zusammensetzung: 83 Vol.-% Kohlenmonoxid, 1 Vol.-% Kohlendioxid, 3 Vol.-% Stickstoff und 0,07 Vol.-% Wasserstoff sowie 11 Vol.-% Butene zu. Die Carbonylierung verläuft bei einer Temperatur von 135°C und unter einem Druck von 650 bar. Das am Kopf des Hochdruckgefäßes abgenommene Produkt wird entspannt und das abgetrennte Gas wiederum in die Synthese zurückgeführt. Anschließend werden die überschüssigen $C_4$-Kohlenwasserstoffe abdestilliert. Sie enthalten noch 1200 ppm nicht umgesetztes Butadien. Der Umsatz bezogen auf Butadien beträgt 99,8 %, die Selektivität zu Pentensäuremethylester beträgt 92 %. Die Raumzeitausbeute beträgt 0,08 kg Pentensäuremethylester je Liter Reaktionsraum und Stunde.

Vergleichsbeispiel

Man verfährt wie in Beispiel 1 beschrieben. Dem Reaktionsgefäß werden stündlich 22 Gew.-Teile $C_4$-Schnitt mit 41 % (m/m) Butadien-1,3, 18,6 Gew.-Teile 3-Methylpyridin, 7,3 Gew.-Teile Methanol, 0,67 Gew.-Teile Cobalt in Form von Coblatcarbonyl und 18,0 Gew.-Teile eines Gasgemisches folgender Zusammensetzung, 83 Vol.-% Kohlenmonoxid, 1 Vol.-% Kohlendioxid, 3 Vol.-% Stickstoff, 0,07 Vol.-% Wasserstoff sowie 12 Vol.-% Butene zugeführt. Die Umsetzung erfolgt wie in Beispiel 1 beschrieben. Der Umsatz bezogen auf Butadien beträgt 99,8 %, die Selektivität zu Pentensäuremethylester beträgt 92 %. Die Raumzeitausbeute beträgt 0,067 kg Pentensäuremethylester je Liter Reaktionsraum und Stunde.

**Patentansprüche**

1.  Verfahren zur Herstellung von Pentensäurealkylestern durch Umsetzung von Butadien mit Kohlenmonoxid und Alkanolen in Gegenwart von Cobaltcarbonylkomplexen und tertiären, aromatischen heterocyclischen Stickstoffbasen bei einer Temperatur von 80 bis 160°C und unter einem Druck von 100 bis 1000 bar, dadurch gekennzeichnet, daß man 0,1 bis 10 mol-% Wasser, bezogen auf eingesetztes Butadien mitverwendet.

2.  Verfahren nach Anspruch 1, dadurch gekennzeichnet, daß man 0,5 bis 8 mol-% Wasser bezogen auf Butadien mitverwendet.

3.  Verfahren nach den Ansprüchen 1 und 2, dadurch gekennzeichnet, daß man die Umsetzung in 2 bis 4 hintereinander geschalteten Stufen durchführt.

4.  Verfahren nach den Ansprüchen 1 bis 3, dadurch gekennzeichnet, daß man Wasser in der ersten und/oder den nachfolgenden Stufen zugibt.

5.  Verfahren nach den Ansprüchen 1 bis 4, dadurch gekennzeichnet, daß man eine Temperatur von 100 bis 150°C einhält.

**6.** Verfahren nach den Ansprüchen 1 bis 5, dadurch gekennzeichnet, daß man einen Druck von 120 bis 700 bar einhält.

**7.** Verfahren nach den Ansprüchen 1 bis 5, dadurch gekennzeichnet, daß man 3-Methylpyridin oder 4-Methylpyridin oder deren Gemische verwendet.

**8.** Verfahren nach den Ansprüchen 1 bis 7, dadurch gekennzeichnet, daß man Butadien enthaltenden $C_4$-Schnitt verwendet.

## Claims

**1.** A process for preparing an alkyl pentenoate by reacting butadiene with carbon monoxide and an alkanol in the presence of a cobalt carbonyl complex and a tertiary aromatic heterocyclic nitrogen base at from 80 to 160° C and from 100 to 1,000 bar, which comprises also using from 0.1 to 10 mol % of water, based on butadiene used.

**2.** A process as claimed in claim 1, wherein from 0.5 to 8 mol % of water, based on butadiene, is present.

**3.** A process as claimed in claim 1 or 2, wherein the reaction is carried out in from 2 to 4 consecutive stages.

**4.** A process as claimed in any of claims 1 to 3, wherein water is added in the first and/or the subsequent stages.

**5.** A process as claimed in any of claims 1 to 4, wherein a temperature of from 100 to 150°C is maintained.

**6.** A process as claimed in any of claims 1 to 5, wherein a pressure of from 120 to 700 bar is maintained.

**7.** A process as claimed in any of claims 1 to 5, wherein 3-methylpyridine or 4-methylpyridine or a mixture thereof is used.

**8.** A process as claimed in any of claims 1 to 7, wherein a butadiene-containing $C_4$ cut is used.

## Revendications

**1.** Procédé de préparation de penténoates d'alkyle par la réaction du butadiène avec le monoxyde de carbone et des alcanols, en présence de complexes du cobaltcarbonyle et de bases azotées tertiaires, hétérocycliques, aromatiques, à une température de 80 à 160°C et sous une pression de 100 à 1000 bars, caractérisé en ce que l'on utilise conjointement de 0,1 à 10% molaires d'eau, par rapport au butadiène mis en oeuvre.

**2.** Procédé suivant la revendication 1, caractérisé en ce que l'on utilise conjointement de 0,5 à 8% molaires d'eau, par rapport au butadiène.

**3.** Procédé suivant l'une quelconque des revendications 1 et 2, caractérisé en ce que l'on entreprend la réaction en 2 à 4 étapes successives.

**4.** Procédé suivant les revendications 1 à 3, caractérisé en ce que l'on ajoute de l'eau au cours de la première et/ou des étapes suivantes.

**5.** Procédé suivant l'une quelconque des revendications 1 à 4, caractérisé en ce que l'on maintient une température de 100 à 150°C.

**6.** Procédé suivant l'une quelconque des revendications 1 à 5, caractérisé en ce que l'on maintient une pression de 120 à 700 bars.

**7.** Procédé suivant l'une quelconque des revendications 1 à 5, caractérisé en ce que l'on utilise la 3-méthylpyridine ou la 4-méthylpyridine, ou leurs mélanges.

8. Procédé suivant l'une quelconque des revendications 1 à 7, caractérisé en ce que l'on utilise une coupe en $C_4$ contenant du butadiène.